Europäisches Patentamt

(19) European Patent Office

Office européen des brevets

(11) Publication number: 0 145 173
A1

(12) # EUROPEAN PATENT APPLICATION

(21) Application number: 84307093.9

(22) Date of filing: 16.10.84

(51) Int. Cl.⁴: A 61 N 1/42
A 61 B 5/02, A 61 B 8/06

(30) Priority: 17.10.83 US 542677

(43) Date of publication of application:
19.06.85 Bulletin 85/25

(84) Designated Contracting States:
AT BE CH DE FR GB IT LI LU NL SE

(71) Applicant: ELECTRO-BIOLOGY, INC
300 Fairfield Road
Fairfield New Jersey 07006(US)

(72) Inventor: Pratt, George W.
67 Glezen Lane
Wayland Massachusetts 01778(US)

(72) Inventor: Koblik, Philip D.
14 Oak Street
Grafton Massachusetts 01519(US)

(74) Representative: Wright, Peter David John et al,
R.G.C. Jenkins & Co. 12-15, Fetter Lane
London EC4A 1PL(GB)

(54) Modification of blood-circulatory dynamics within a living body.

(57) The invention contemplates a method and apparatus for non-invasively and locally enchancing the presence of blood and/or flow of blood in a selected region (10) of a living body by means of external application of an electromagnetic coil (11,12) connected to a signal generator (13). Techniques of monitoring the blood for desired change in the selected region are also described using transducer means (15) for producing an electric signal.

FIG. I.

MODIFICATION OF BLOOD-CIRCULATORY
DYNAMICS WITHIN A LIVING BODY

BACKGROUND OF THE INVENTION

This invention relates to the modification of the interaction of living tissues and/or cells with charged species in their environment, to bring about a change in blood-circulatory dynamics within a living body.

Ryaby, et al., US Patent Nos. 4,105,017, 4,266,532 and 4,266,533 describe means for effecting surgically non-invasive direct inductive coupling to an afflicted body region, whereby one or more induced electric voltage pulses and concomitant current signals conform to a highly specific pattern and have been found to develop therapeutically beneficial treatment of the afflicted region, as for example in the enhancement of repair of bone fractures, non-unions, and the like. In general, the involved treatment head or heads have comprised one or more large coils, which have served well for the treatment of large-member bones, as in leg regions.

We have discovered that pulsed magnetic fields, as generated by equipment of the character indicated, can also be the means of modifying blood-circulatory dynamics within the body.

## BRIEF STATEMENT OF THE INVENTION

It is an object of the invention to provide a method and means for predictably and selectively modifying blood-circulatory dynamics within a living body.

Another object is to achieve the above object in a selected local region of the body.

A further object is to provide means whereby a given and largely predetermined change in blood circulation can be initiated, monitored and controlled.

The invention achieves these objects by so employing an electrical coil, which may be a loop comprising bundled multiple turns, that the area bounded by the loop substantially embraces or conforms to a lateral projection of a selected local body region, in which region it is desired to locally enhance or otherwise affect blood-circulatory dynamics. The area bounded by the loop is non-invasively positioned adjacent the involved body part, with the loop area in general register with such lateral projection. Pulsed electrical signals, which may be but are not necessarily those as described in said Ryaby, et al. patents, are applied to excite the coil, for a length of time and in a manner to produce a specific blood-circulation change. Illustratively, as will later be explained in greater detail, a 14-minute exposure to such excitation is found to produce an enhanced presence of blood in the region affected by the pulsed magnetic field of the coil, the enhanced presence being observed as at least a 2 percent increase in ultrasonic velocity in the region; upon terminating the excitation, a period of about 30 minutes was required for return to the pre-treatment condition of lesser ultrasonic velocity. Such observations have been confirmed by other techniques of measuring blood presence and/or blood flow in the region exposed to pulsed magnetic-field action, and the measurement may be used as an element of feedback for control of coil excitation.

Thus far, the enhanced flow of blood in a treated region has been the result of using certain pulse criteria, and there is some indication that certain of the waveforms can cause a reduction in blood circulation. Thus, a vaso-dilation or vaso-constrictive action may depend on waveform and "operating point". Monitoring methods, as by use of ultrasonic, thermographic or optical means, are of value in determining the operating point.

DETAILED DESCRIPTION

The invention will be illustratively described in detail, in conjunction with the accompanying drawings, in which:

Fig. 1 is a simplified view schematically showing components in application to a body member in use of the invention;

Figs. 2, 3 and 4 are simplified views to show alternative means of detecting changes in blood dynamics within the body member, for use in place of the detecting components of Fig. 1; and

Fig. 5 is a succession of graphs, in explanation of data pertaining to the invention.

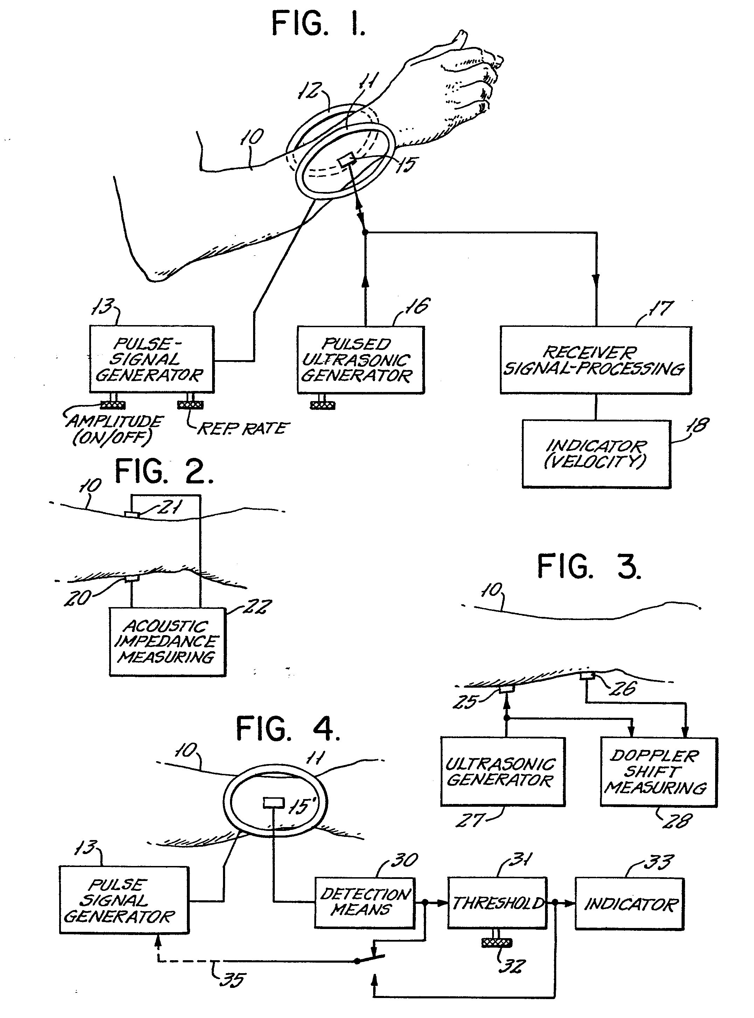

Referring initially to Fig. 1, the invention is shown in application to a portion of a human forearm 10 wherein a localized longitudinally central region is to be subjected to modification of blood-circulatory dynamics, e.g., locally enhanced blood flow, as in support of local surgery and/or of the post-operative healing process. A pulsed magnetic field is established through the local region, by reason of two like electrical coils 11-12, positioned on opposite sides of the limb 10. Each of the coils 11-12 may be a bundle of multiple turns, suitably about 56 turns of B&S gauge-28 coated copper wire, initially wound to a diameter of 23-cm and then bundled and deformed to the generally oval shape shown. Each of the coils 11-12 encompasses an included area approximating the involved lateral projection of the local limb region of pulsed magnetic-field exposure, and the coils are in substantial register with said projection and with each other. Coils 11-12 are interconnected in flux-aiding relation, so that when excited by the output of a pulse generator 13, they establish a substantially uniform distribution of flux development in the included geometric volume defined by and between the coils.

The signal generator 13 and the character of signals produced thereby have been described in said Ryaby, et al. patents, so that further description thereof is not now needed. Provision is shown in generator 13, at 14 and 15, respectively, for selective adjustment of amplitude (including ON/OFF control) of pulse-signal output, pulse shape and for selective adjustment of pulse-repetition rate.

- 5 -

It has been generally indicated above that a relatively short application of pulsed magnetic-field energy at the region localized by coils 11-12 can measurably enhance the flow to and presence of blood within the localized region; and there is some indication that after a prolonged period of pulsed magnetic-field action, the flow enhancement achieves a plateau, and that such action, if further continued, can cause a decrease of flow enhancement.

A variety of means and techniques may be drawn upon to monitor the dynamics and the quantum of the resulting change. In Fig. 1, the monitored quantity is change in ultrasonic velocity, by reason of ultrasonically exciting a transducer 15 in contact with skin within the included area of one (11) of the coils, a generator 16 of pulsed ultrasonic signals being connected to transducer 15 for the purpose. Associated componentry is shown to include a receiver 17 with signal-processing means whereby an indicator 18 may display ultrasonic velocity, as derived from travel time for pulses reflected from bone within limb 10. As previously indicated, at least a 2 percent increase in ultrasonic velocity is thus observable for about a 14-minute exposure to the pulsed magnetic field.

Fig. 2 provides a schematic indication of the use of two ultrasonic transducers 20-21 applied to opposite sides of limb 10 (again within the localized region between coils) in conjunction with suitable means 22 of measuring changes in acoustic impedance. Observed changes in acoustic impedance are another indicator of changes in blood presence in the localized region, acoustic-impedance changes being generally proportional to ultrasonic-velocity changes.

Fig. 3 provides a schematic indication of the use of two ultrasonic transducers 25-26 at longitudinally spaced locations along the same side of limb 10, whereby signal output from an ultrasonic generator 27 may be observed at 28 for changes in Doppler-effect frequency shift attributable to changes in blood flow in the region of pulsed magnetic-field exposure.

The detecting techniques involved in the different embodiments of Figs. 1, 2 and 3 will be understood to be merely illustrative, in that other techniques (including optical-transmission techniques) are or may become available, for noting the quantum of blood-presence change or blood-flow changes. And it will be understood that by having the surgeon or a technician monitor for a desired change, it can be known when to reduce the level of magnetic-pulse generation, or to terminate the same, as in order to remove all obstacles for performance of a surgical operation, it being noted that, after a termination of pulsed magnetic-field exposure, a period of about 30 minutes exists within which to expect enhanced blood presence in the localized region of interest.

Fig. 4 illustrates that any of the described apparatus lends itself to a measure of automation, and therefore a single transducer 15' within coil 11 will be understood to schematically represent the transducer system of any of the embodiments of Figs. 1, 2 or 3. By the same token, in Fig. 4, the detection means 30 will be understood to be as appropriate to the particular selected detection technique for monitoring for change in blood presence or blood flow, and a threshold circuit 31 with means 32 to select a predetermined threshold may therefore provide an output to a suitable indicator 33 when the detected change in blood condition achieves the preset threshold level. The indicator 33 may of course provide a simple warning of achievement of the preset threshold, as by flashing lamp or audible alarm.

Fig. 4 provides further schematic indication of a feedback connection 35 which includes a selector switch 36, whereby for example in the switch position shown the output signal of detection means 30 may provide control of the output of pulse generator 13; in the alternative position of switch 36, the achievement of the preset threshold condition, manifest in the output signal of circuit 31, will be understood to be the means of automatic termination of generator-13 output to coil 11.

Fig. 4 will be still further understood to illustrate that, although it is presently preferred to employ two coils 11-12 as discussed in connection with Fig. 1, the described blood-control effects may also be achieved using but one such coil. The primary difference between one-coil and two-coil usage is in the degree of uniformity and depth of body penetration of the involved pulsed magnetic field.

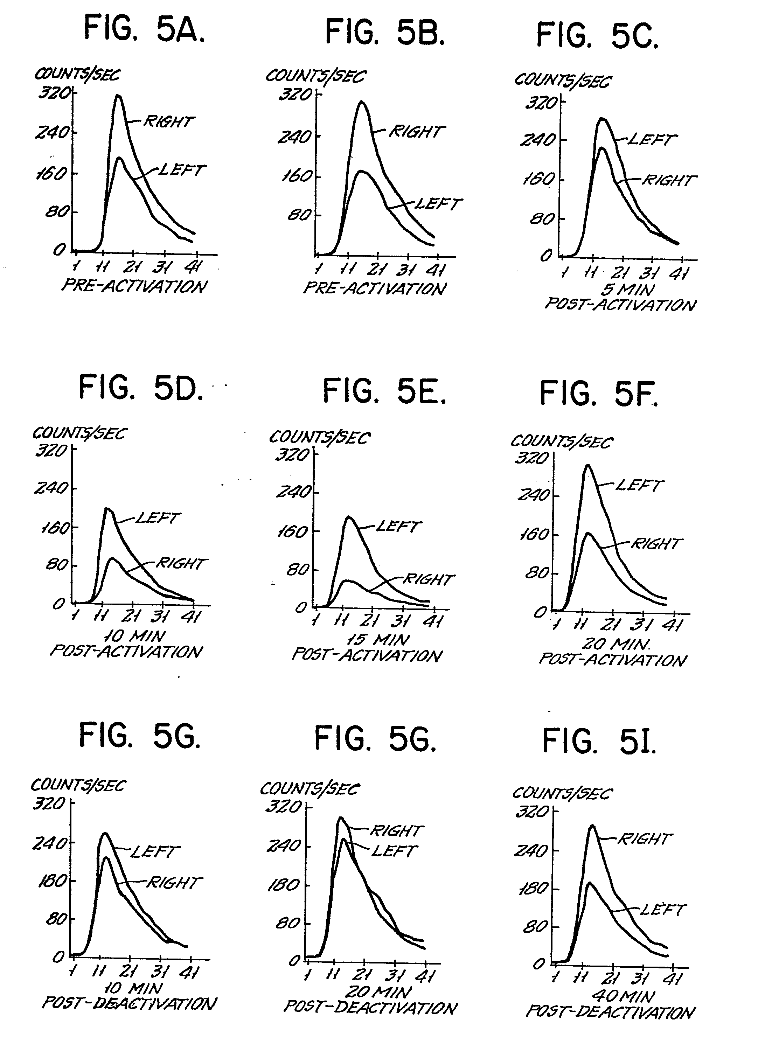

The following example, in connection with the graphs A to I of Fig. 5, will illustrate application of gamma-ray tracer techniques as a means of quantitatively observing the ability of pulsed magnetic fields to enhance blood presence in the locality of field exposure, as compared to a corresponding but remote locality having insignificant field exposure. A dog is anesthetized and restrained so that the front legs are imaged by a gamma camera, with separate provision for simultaneously measuring detected gamma levels in the respective legs at a given time. A first pair of electrical coils 12-13 is strapped to opposite sides of one front leg, and a second pair of like coils is similarly strapped to the other front leg. The left coil pair is excited in the manner described above, while the other pair of coils is not excited and is therefore a dummy.

Fig. 5 is a succession of graphical recordings of measured gamma levels, in each of the respective legs of the dog, at different times, and each curve carries legend identification of the applicable leg. For each of the successive recordings, the recording was made over the same period of time (40 seconds) following injection of radioactive-tracer solution, the latter having been selected for a short (e.g., 5 seconds) half-life.

Fig. 5A depicts an at-rest condition, prior to which there has been no coil excitation and, therefore, no pulsed magnetic-field exposure of either leg. The ratio of areas beneath the respective curves is 1.52:1, the predominance of blood being detected in the right. leg, for reasons which can only be presumed to be physical, as for example, the dog having normally always preferen-

- 9 -

0145173

tially favored primary reliance on his right leg. The curves of Fig. 5B were recorded 5 minutes later, as a check on conditions prior to magnetic-pulse excitation; the measured areas beneath the curves are in the ratio 1.63:1, again favoring the right leg, and substantially confirming the measurements of Fig. 5A.

Having thus recorded the at-rest condition, the coils on the left leg were excited, in the manner described for Fig. 1 and to the exclusion of the dummy coils on the right leg, thus exposing the left leg to activation by pulsed magnetic fields. At 5 minutes past commencement of activation, a further isotope injection and recording was made, accounting for the curves of Fig. 5C. In Fig. 5C, the predominance of blood presence is seen to have shifted, from the unexposed right leg to the exposed left leg, and the measured ratio of right-leg/left-leg blood presence is 0.77:1.

The injection and recording process was repeated at 10 minutes, 15 minutes and 20 minutes after commencement of activation, with recorded measurements as depicted in Figs. 5D, 5E and 5F, respectively. Area measurements beneath the curves of these recordings yield right-leg/left-leg blood-presence ratios of 0.50:1, 0.33:1, and 0.54, respectively. Apparently, response to continued activation peaks after about 15 minutes, by which time the presence of blood in the unfavored left leg (as compared to the favored right leg) has increased by a factor of about 5 times.

Having made the recording for Fig. 5F, coil-excitation was terminated, and the injection/measurement cycle was repeated at successive intervals which were 10 minutes, 20 minutes and 40 minutes post-deactivation of the coils.  The recordings of Figs. 5G, 5H and 5I, respectively, were made at end of these intervals, showing right-left/left-leg blood-presence ratios of 0.81:1, 1.18:1, and 1.65:1, the last of which will be recognized as certifying that the dog's blood-presence condition has been returned to its original state, per Figs. 5A and 5B.

The conclusion is clear that local predominance of tracer presence means local presence of blood presence. The pulsed magnetic fields have an effect akin to vaso-dilation, and they may be operative in the recruitment of capillaries in the localized region of body exposure.

The pulsed magnetic-field signals which evoked the data reported in connection with Fig. 5 can be described as repeated bursts of an asymmetrical pulse cycle wherein, for each pulse, a first-polarity pulse portion is of relatively small amplitude and long duration (e.g., 250 microseconds) and an opposite-polarity pulse portion is of relatively large amplitude and short duration (e.g., 9 microseconds); maximum pulse amplitude is 1.6 millivolts*; burst duration is 30 milliseconds, burst repetition rate is 2 Hz, and the period between pulses of a burst is 22 microseconds.  These pulse data will be seen to accord with the low-power signal data specified for light-weight battery-powered portable equipment described in Ryaby, et al. pending application Serial No. 413,539, filed

---

\* As measured by the probe technique described in said Ryaby, et al. patents.

August 31, 1982, and in subsequent blood-flow measurements on human beings, it has been confirmed that the same pulsed-magnetic signals produce enhanced blood-flow in humans, the measurements being made without resort to radioactive materials, using a laser/Doppler technique.

Further experiments suggest that certain fields may produce a result akin to vasoconstriction and that the effect of the pulsed magnetic fields on blood-circulatory dynamics depends on the initial physiological condition at the time of implementation of the pulsed fields.

While the invention has been described for various illustrative embodiments, it will be understood that modifications may be made without departure from the scope of the invention.

CLAIMS:

1.  The method of locally affecting blood circulation in a selected region of a living body, which comprises selecting an electromagnetic coil of included inner area substantially matching the area of a lateral projection of the selected region, positioning the coil adjacent the body in substantial register with said projection, whereby the selected region is within at least part of the magnetic field produced by the coil when electrically excited, exciting the coil with an electric signal capable of modifying the interaction of living cells and/or tissues with charged species within the magnetic field generated by the coil, monitoring change in a blood condition within the selected region, and utilizing the monitored change to determine a modification of the excitation of the coil.

2.  The method of claim 1, in which the monitoring step includes non-invasive wave propagation through a part of the selected region, and evaluating change in speed of such propagation.

3.  The method of claim 2, in which the wave propagation is ultrasonic.

4. The method of claim 3, in which the propagation is directed to a bone in the selected region, and speed change is observed by echo-ranging referenced to the bone.

5. The method of claim 2, in which the evaluation includes non-invasive detection or propagated waves at a body location within the selected region but at offset from the location of wave propagation, and observing for Doppler shift in the output of such detection.

6. The method of claim 1, in which the selected coil is one of two like coils connected in flux-aiding relation and positioned in spaced relation adjacent opposite sides of the selected body region, with the respective axes of said coils in general alignment and passing through the selected region.

7. The method of claim 1, in which the coil excitation includes a pulsating signal component.

8. The method of claim 1, in which the coil excitation is by a succession of asymmetrical pulses.

9. The method of selectively modifying blood-circulatory dynamics in a living body, which comprises exposing at least part of the body to a pulsating electromagnetic field.

10. The method of claim 9, wherein the exposure is for a predetermined period of time.

11. In combination, an electromagnetic coil adapted for external application to a selected local region of a living body, a signal generator connected to excite said coil with a signal capable of modifying living cell and/or tissue behavior within the magnetic field generated by said coil, and transducer means adapted for body application at the selected region and producing an electric-signal output responsive to a change in blood-circulatory dyanamics.

12. The combination of claim 11, including a feedback-control connection from said transducer means to said signal generator.

# FIG. I.

| 13 | 16 | 17 |
|---|---|---|
| PULSE-SIGNAL GENERATOR | PULSED ULTRASONIC GENERATOR | RECEIVER SIGNAL-PROCESSING |

AMPLITUDE (ON/OFF)    REP. RATE

INDICATOR (VELOCITY) 18

# FIG. 2.

ACOUSTIC IMPEDANCE MEASURING 22

# FIG. 3.

| ULTRASONIC GENERATOR | DOPPLER SHIFT MEASURING |
|---|---|
| 27 | 28 |

# FIG. 4.

| 13 | 30 | 31 | 33 |
|---|---|---|---|
| PULSE SIGNAL GENERATOR | DETECTION MEANS | THRESHOLD | INDICATOR |

32

35

0145173

FIG. 5A.

COUNTS/SEC

RIGHT
LEFT

PRE-ACTIVATION

FIG. 5B.

COUNTS/SEC

RIGHT
LEFT

PRE-ACTIVATION

FIG. 5C.

COUNTS/SEC

LEFT
RIGHT

5 MIN
POST-ACTIVATION

FIG. 5D.

COUNTS/SEC

LEFT
RIGHT

10 MIN
POST-ACTIVATION

FIG. 5E.

COUNTS/SEC

LEFT
RIGHT

15 MIN
POST-ACTIVATION

FIG. 5F.

COUNTS/SEC

LEFT
RIGHT

20 MIN.
POST-ACTIVATION

FIG. 5G.

COUNTS/SEC

LEFT
RIGHT

10 MIN
POST-DEACTIVATION

FIG. 5G.

COUNTS/SEC

RIGHT
LEFT

20 MIN
POST-DEACTIVATION

FIG. 5I.

COUNTS/SEC

RIGHT
LEFT

40 MIN
POST-DEACTIVATION

# PARTIAL EUROPEAN SEARCH REPORT

**0145173**

Application number

European Patent Office

which under Rule 45 of the European Patent Convention shall be considered, for the purposes of subsequent proceedings, as the European search report

## DOCUMENTS CONSIDERED TO BE RELEVANT

EP 84307093.9

| Category | Citation of document with indication, where appropriate, of relevant passages | Relevant to claim | CLASSIFICATION OF THE APPLICATION (Int. Cl.4) |
|---|---|---|---|
| X | AT - B - 359 178 (RODLER)<br>* Page 2, lines 1-6, 35-37, 43-53; page 6, lines 1-17; pages 8,9; claim 1 *<br>-- | 11 | A 61 N 1/42<br><br>A 61 B 5/02<br><br>A 61 B 8/06 |
| X | DE - A1 - 2 707 574 (GÖDDE)<br>* Pages 1,2; claims 1,2; page 11, line 5 - page 12, line 10; fig. 1 *<br>-- | 11,12 | |
| X | DE - A - 2 255 757 (DUROUX)<br>* Page 14, claims 1,2; fig. 1, 2 *<br>-- | 11 | |
| Y | EP - A1 - 0 048 451 (DROLET)<br>* Abstract; page 11, line 16 - page 13, line 13; page 47; claim 1 *<br>-- | 11 | TECHNICAL FIELDS SEARCHED (Int. Cl.4)<br><br>A 61 N<br><br>A 61 B |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 11,12
Claims searched incompletely: —
Claims not searched: 1-10
Reason for the limitation of the search:

According to Art. 52(4) EPÜ

| Place of search | Date of completion of the search | Examiner |
|---|---|---|
| VIENNA | 27-02-1985 | NEGWER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

  & : member of the same patent family, corresponding document

EPO Form 1505.1. 03.82

European Patent Office

**PARTIAL EUROPEAN SEARCH REPORT**

EP 84307093.9

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| Y | DE - B1 - 2 911 258 (CHMIEL) <br><br> * Column 1, claim 1; column 2, line 43 - column 3, line 22 * <br><br> -- | 11 | |
| X | US - A - 2 667 159 (GOLDBERG) <br><br> * Column 1, lines 1-39; column 7, claim 1; fig. 1,6 * <br><br> -- | 11 | |
| A | DE - A1 - 3 041 965 (AIMANTE) <br><br> * Page 2, paragraph 1,2; page 6, lines 12-20; page 7, line 21 - page 8, line 10 * <br><br> ---- | 11 | TECHNICAL FIELDS SEARCHED (Int. Cl.4) |